Europäisches Patentamt

European Patent Office

Office européen des brevets

(11) Publication number: **0 391 852**
**A2**

# (12) EUROPEAN PATENT APPLICATION

(21) Application number: 90810255.1

(22) Date of filing: 29.03.90

(51) Int. Cl.⁵: **A61K 9/02, A61K 47/30, A61K 47/32, A61K 47/36, A61K 47/38, A61K 47/40**

(30) Priority: 03.04.89 CH 1199/89

(43) Date of publication of application:
10.10.90 Bulletin 90/41

(84) Designated Contracting States:
DE FR GB IT

(71) Applicant: WARNER-LAMBERT COMPANY
201 Tabor Road
Morris Plains New Jersey 07950(US)

(72) Inventor: Hagenlocher, Martin, Dr.
Sportplatzstrasse 4
D-8044 Lohhof(DE)
Inventor: Moell, Friedrich
Im Grundhof 6
CH-4600 Olten(CH)
Inventor: Speiser, Peter, Prof.
Freudenbergstrasse 101
CH-8044 Zuerich(CH)

(74) Representative: Silbiger, Jakob, Dr.
c/o CAPSUGEL AG Fabrikmattenweg 2-4
CH-4144 Arlesheim(CH)

(54) **Pharmaceutical compositions.**

(57) A composition for rectal or vaginal application of drugs, characterised in that the composition is substantially free of fat and contains, together with the effective substance or effective substances and possibly further carrier substances, at least one hydrocolloid, which, through interaction with local body fluid, effects disintegration of the applied formulation.

EP 0 391 852 A2

## Pharmaceutical composition

The present invention relates to a new, substantially fat free pharmaceutical composition, which is particularly suited for rectal or vaginal application in pharmaceutical capsules.

A plurality of medicines are suitable for rectal or vaginal therapy with the aim of making the medicines absorb systemically or in order to achieve an effect locally in the rectum or the vagina. Known forms of medicines for this are suppositories, micro-or mini-enemas, rectal- and vaginal tablets as well as some further specialised forms.

Suppositories which either use lipophilic basic materials such as fats, or hydrophilic basic materials such as polyethylene glycols, as carriers, are the most common forms of medicine for rectal application. Their manufacture occurs either by dispersion or dissolving the effective substances and auxiliary agents in the melted basic material or by compressing effective substances and auxiliary agents, wherein the carrier substances must exist in granulated or pulverized form. The finished suppositories must have a sufficient breaking strength, in order to be able to be applied by the patient.

Suppositories have, however, numerous disadvantages. The hardening of hard fats usually leads to the raising of their melting point to above the body temperature and thus to the loss of effect of the pharmaceutical formulation. Since the body temperature fluctuates considerably in an inter- and intra-individual manner, even with suppositories with a melting point of 37° C variations in bioavailability can be expected. For producing suppositories only a limited number of auxiliary agents is available since suppositories must, on the one hand, have a melting point which is as high as possible, so as not to melt when being transported or during application, but which, on the other hand, melt below 37°C after application or must dissolve in the small amount of rectal fluid, in order to enable a release of the effective substance or of the effective substances. In general the manufacture of suppositories occurs by distribution of the effective substances in the melted mass, which, particularly with temperature-sensitive effective substances such as polypeptides, is linked with stability problems. Furthermore, suppositories can melt in the hand or even break during application. Even mechanical strength can suffer in storage.

When using polyethylene glycols or polyethylene glycol derivatives, such as PEG-23-laurates as carrier mass for rectal forms of medicine, considerable damage to the rectal mucous membrane can occur, as is for example described by E.M. Holyhead et al , Br. J. exp. Path (1983). 64, 456f, or by N.W. Thomas. et al, in Intern. Journal of Pharmaceutics, 44 (1988) 261-263.

Common to the known formulations is that they distribute in melted or dissolved form over the rectal mucous membrane after disintegration of the pharmaceutical formulation in the rectum or in the vagina. This leads mainly to a relatively quick release and, in the case of systemic therapy to a quick absorption of the effective substances (= active ingredient(s)). For many effective substances, however, a delayed release is desired, on the one hand to avoid high initial blood level values, and on the other hand to lengthen the required application intervals with substances with a short half-life period. Whilst such forms of medicine for delayed release of the effective substance for peroral formulations have been known for a long time, no formulations have hitherto existed which, after a rectal or vaginal dose, enable a delayed release of the effective substance over several hours and which at the same time do not have the above-mentioned disadvantages.

It has now been discovered that not all of these disadvantages occur if, in accordance with the invention, a solid composition for rectal or vaginal application is used, which is substantially free of fat and, together with the drug, contains at least one hydrocolloid, which through absorption of the body fluid swells and initiates the disintegration of the applied formulation and at the same a controlled effective substance release is guaranteed.

The present invention relates to a composition for rectal or vaginal application of drugs, characterised in that this composition is substantially free of fat and contains, together with the effective substance or effective substances and possibly further carrier substances, at least one hydrocolloid, which, through interaction with the local body fluid, effects a controlled release of the applied formulation. The effectiv form is preferably substantially free of water.

The composition for rectal or vaginal use is, in accordance with the invention, filled into pharmaceutical capsules, such as pharmaceutical hard and soft gelatine capsules, known per se, preferably in hard gelatine capsules, in particular in such two-part capsules, which can be produced by means of the dipping method or, for example those which are produced in the injection-moulding method from hydrophilic polymers, such as starch capsules.

In this sense the invention also relates to capsules, preferably capsules with a hard-casing, in particular those of gelatine or starch, for rectal or vaginal application, which contain a composition in accordance with the invention.

The capsules mentioned have, preferably, a rounded-off form and are coated with a lubricant coat. such lubricant coats are known per se. The capsules can be additionally sealed in a known way, or for two-part hard gelatine capsules, provided with a band, which covers the cap edge and a bordering part of the outer wall of the container part. All popular sizes of hard gelatine capsules can be used.

The composition in accordance with the invention is preferably solid and exists in granular or powder form. No heating of the effective substance is necessary, which has preferably an average particle size of max. 250 μm and preferably of max. 150μm.

The dosage itself is only dependent on the therapeutic aim to be achieved.

The filling for the capsules, or the composition according to the invention, can be produced by simple mixing of the individual components. The release of the effective substance is independent of the body temperature; the filling itself does not need mechanical strength.

Under "substantially free of fat" the understanding in respect of the present invention is that no fat need be used as carrier substance in which the effective substance is dissolved or suspended. As already mentioned, in conventional suppositories the effective substance or effective substances are dissolved or suspended in a cast or compressed matrix of the carrier substance, which represents a hard fat or a solid polyethylene glycol. This is not necessary here, rather the effective substance or substances are only mixed with the swelling material and the further auxiliary agents. It is quite possible also to add to the mixture, in accordance with the invention, lipophilic and/or hydrophilic auxiliary agents, alone or such ones which contain one effective substance in a dissolved or suspended form, or preferably those which contain no effective substance, for example fats or polyethylene glycols in pulverized or powdered form. For this purpose concentrations of up to 30 % are possible, preferably, however, the added concentrations are below 20 % by weight, particularly below 10 % by weight, relative to the total weight of the composition.

As effective substance or effective substances in principle all stable pharmaceutical effective substances can be used, which are used in systemic or local therapy for rectal or vaginal application. These are preferably solid at room temperature, e.g. in the form of a powder or granulated material, or transformed by means of a suitable method, for example DL-alpha-Tocopherol acetate (Vitamin E), which is liquid at room temperature, into a solid form e.g. absorbed onto a solid body (e.g. $SiO_2$), or imbedded into a matrix, e.g. gelatine-carbohydrate matrix.

Effective substances which are suitable for the composition according to the invention and for the filling into pharmaceutical capsules in accordance with the invention, can be soluble or insoluble in water. Examples of these are analeptic agents, e.g. pentetrazole; analgesics, e.g. tilidin-HCl, pentazocine, morphine-HCl, paracetamol, metamizolsodium, acetylsalicylic acid, propyphenazone; anti-emetics e.g. dimenhydrinate, metoclopramide; anti-epileptic agents e.g. phenytoin, valproic acid; anti-histamines e.g. diphenhydramine-HCl; antibiotics e.g. penicillin, cephalosporin, tetracycline, erythromycin; anti-infectants e.g. povidone-iodine; anti-hypertensive agents e.g. nifedipin; anticoagulants e.g. heparin; anti-mycotic agents e.g. amphotericin B, clotrimazole, nystatin; antiviral means e.g. acyclovir; anti-rheumatic agents e.g. indomethacine, Diclofenac sodium, ibuprofen; beta-receptor blockers e.g. propranolol; anti-bronchospasm agents e.g. theophylline, aminophylline; chemotherapeutic agents e.g. nitrofurantoin; steroides for local rectal and vaginal treatment e.g. prednisolone, hydrocortisone, triamcinolone; polypeptides e.g. vasopressin, insulin, glucagon, calcitonin, gastrin, human growth hormones, interleukines; hormones e.g. oestrogen, testosterone, prostaglandins; antitussive agents e.g. codeine phosphate; anti-arrhythmia agents e.g. lidocaine; neuroleptic agents, e.g. promethazine, chlorpromazine; spasmolysants e.g. scopolamine butylbromide; tranquilizers e.g. diazepam, temazepam; tuberculostatic agents e.g. isoniazid; cytostatic drugs e.g. Methotrexate, 5-fluorouracil.

Preferred substances for systemic rectal therapy are polypeptides of small to medium molecular weight such as vasopressin, calcitonin, insulin and interleukines; anti-rheumatic agents such as Diclofenac-sodium; analgesics such as paracetamol, morphine and morphine analogues and anti-emetic agents such as metoclopramide.

Preferred substances for local rectal and vaginal therapy are anti-mycotic agents such as clotrimazole, amphotericin-B; anti-infectants such as povidone-iodine and hormones such as estradiol, prednisolone.

The hydrocolloid to be used in accordance with the invention acts as swelling means, as through absorption of body fluid and its enlarged volume due to this, it effects a quick distribution of the composition over the mucous membrane surface and at the same time, depending on the selected quantity and type of the swelling means, guarantees a timed dosed or delayed release of the effective substance.

The composition according to the invention has effect even with a content of 5% hydrocolloid, and it contains preferably at least 10 % by weight and particularly at least 15 % by weight of hydrocolloid relative to the total weight of the composition. In general the composition contains 5 - 99.9% by weight, preferably 10 - 95 % by weight, and particularly 40 -90 % by weight of hydrocolloid relative to the total weight of the composition.

As a hydrocolloid or as a mixture of such hydrocolloids,compounds can be used which, when the aqueous agents enter, with an increase in the volume, quickly swell extensively and completely to form a hydrogel. The viscosity of à 2% aqueous preparation of the swelling material, measured by means of an Ubbelohde capillary viscometer or Brookfield viscometer at 20 ° C, lies below 5000 cps, preferably below 1000 cps.

Preferred swelling materials in respect of the present invention are those which, when carrying out the method of examination "Examining the swelling properties", described further below have after 5 minutes a net swelling volume of at least 1 ml per 300 mg of swelling material.

Swelling materials in respect of the invention are not only those usually used for peroral administration in capsules for achieving a retarding effect, but also those used for achieving a release of effective substance as quickly as possible, i.e. those swelling means used as so-called "bursting means" or "disintegration aiding means" in capsules or tablets or those swelling materials which form gels of low viscosity with water, i.e. those which are not usually able to be used for delaying the release of an effective substance, because the viscosity of their solution is too low, in order to be able to construct a stable diffusion barrier. Typical examples for the first case are modified starches such as sodium carboxymethyl starch (Explotab$^R$) or also inorganic materials such as colloidal silicic acid (Aerosil$^R$). Typical examples for the second case are cellulose derivatives of low viscosity such as Tylose MH 300$^R$ (Hoechst Ag, D-Frankfurt) or Viscontran MHPC 400$^R$ (Henkel KG,D-Düsseldorf).

Swelling materials which are suitable may be natural, synthetic or partially synthetic.

Suitable hydrocolloids are for example types of starch, preferably corn and potato starch, trapa starch; modified or pre-treated starches, preferably carboxylized starches such as carboxymethyl starch (Explotab$^R$, Primojel$^R$), corn swelling starch (Prejel$^R$), Sta-RX-1500$^R$; pectin derivatives, preferably ultraamylopectin (Amijel$^R$), beta-amylose; galactomannane, such as St, John's bread grain flour (e.g. Meyprodyn$^R$), guar (Meypro-guar$^R$, Meyprogate$^R$ etc.), konjacamannane; other swellable polysaccharides, xanthines (Keltrol F$^R$), dextrans, hyaluronic acids; cellulose, cellulose esters and cellulose ethers; preferably sodium carboxymethyl cellulose, hydroxypropyl cellulose, hydroxypropylmethyl cellulose, methyl cellulose, ethyl cellulose, hydroxyethyl cellulose, hydroxyethylmethyl cellulose, wherein those qualities are preferred which, with water give solutions with a low viscosity or hydrogels; alginic acids and alginic acid salts such as sodium and calcium alginates, alginic acid derivatives such as propylene glycol alginates; inorganic hydrocolloids such as colloidal silicon dioxide (Aerosil$^R$, Cab-O-Sil$^R$), bentonites (Veegum$^R$); agar-agar, tragacanth, rubber arabicum, Sterkulia rubber carrageenan formaldehydecasein (Esma-Spreng$^R$; Geletol$^R$); synthetic, swellable polymers such as polyacrylic acids, polymethacrylates, hydroxyethylmethacrylate and their derivatives, or mixtures of such hydrocolloids.

Examples of this are : carboxymethyl starch (Explotab$^R$, Primojel$^R$), sodium-carboxymethyl cellulose, hydroxypropylmethyl cellulose, methyl cellulose, salts of polyacryl acids or a mixture of such combinations.

The composition according to the invention can contain, together with (a) the effective substance and (b) the hydrocolloid, further auxiliary materials for optimization, for example:

(c) flow regulating means, e.g. talcum in concentration typically of < 4%

(d) lubricant, e.g. magnesium stearate, talcum; glycerolbehenate (Compritol 288, Gattefosse)

(e) fillers, e.g. lactose,

(f) carrier substances, e.g. polyethylene glycols or fats, in small proportions

(g) wetting agents e.g. sodium lauryl sulphate and possibly further auxiliary agents, which improve stability, processibility or bioavailability.

A typical composition has the following ingredients with weights in percentages.

| (a) Effective substance | 0.0001 % - 80 % |
|---|---|
| (b) Hydrocolloid | 20 % - 90 % |
| (c) Flow regulating means | 0.2 % - 4 % |
| (d) Lubricant | 0.5 % - 4 % |
| (e) Filler | 0 % - 70 % |
| (f) Carrier substances | 0 % - 10 % |
| (g) Wetting agent | 0.1 % - 5 % |

A simple composition can be obtained for example if a finely-powdered effective substance (particle size smaller than 125 μm) is mixed in a dosage of 200 mg or less with 200 mg sodium carboxymethyl starch (Primojel, Avebe, NL-Foxhol).

If the dosage of the effective substance lies below 200 mg, then lactose can supplement it. This mixture can be filled into capsules of size 0, elongated and be provided with a three-layered coating (Moell et al, Stability of gliding coats for rectal hard gelatin capsules, Acta pharma. techn. 34, 24S (1988) as a lubricant coat.

For the manufacture of the composition the individual components are intensively mixed. The mixture obtained can then be filled into capsules in a known manner. Naturally it is possible to heat up the mixture easily, in as far as this is considered to be useful.

The filled capsules obtained can be coated with a lubricant coat, as described for example in A.M. Hannula et al, Evaluation of a lubricant coat for rectal hard gelatin capsules, Acta pharmaceutica techn., 32, 26, 1986.

The following listed examples illustrate the invention.

Examination of the swelling properties of the hydrocolloid

Swelling properties.

300 mg of the finished mixture or of the swelling material alone are placed in a graduated test tube and the volume is read. The test tubes are thermoequalised in a water bath for 15 minutes. Then by means of a pipet 5 ml of the following buffer is carefully added:

| | |
|---|---|
| $NaH_2 PO_4 . 2H_2O$ | 10.40 g |
| $Na_2 HPO_4 . 2H_2o$ | 47.48 g |
| NaCl | 23.00 g |
| $H_2O$ dest. ad | 5000 ml |
| set to pH | 7.38 |

The time dependent increase in volume of the gel which is forming is measured and whether the mixture has completely swollen is judged visually.

**Release of the effective substance in vitro.**

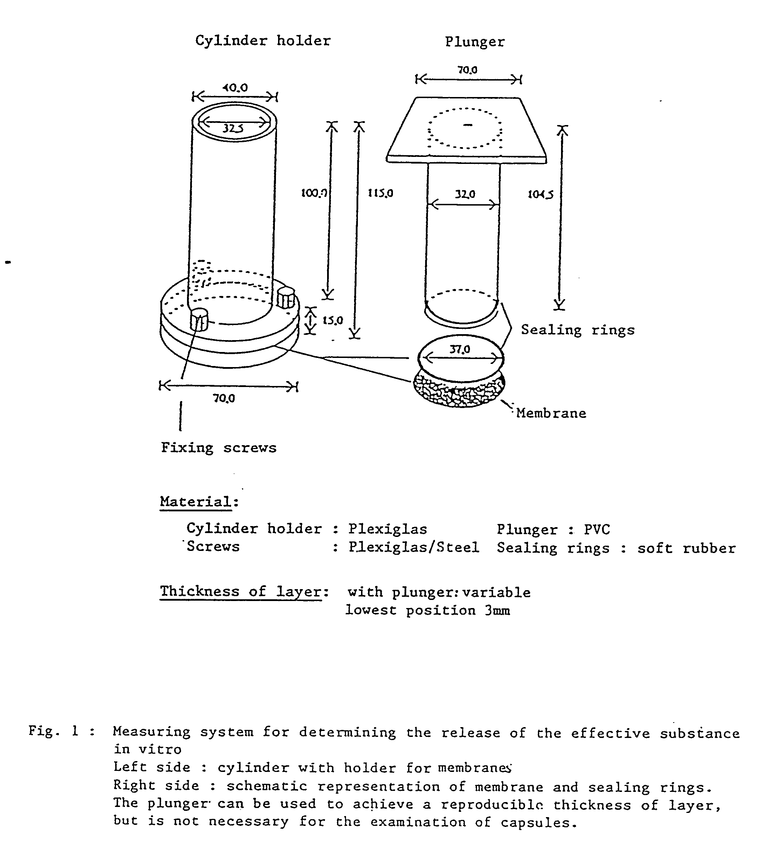

Figure 1 shows apparatus for determining the release of the effective substance in vitro. The measuring system is mounted in such a manner over a beaker (volumetric capacity e.g. 1500 ml), such that the membrane has contact exactly with the surface of the releasing medium and is located in the centre of the beaker. The intermixing of the releasing medium occurs through a magnetic stirrer; the number of revolutions is adjusted to a fixed region (usually between 100 and 200 revolutions per minute). The whole measuring system is thermoequalized for approximately 60 min. At the time t = 0 a capsule is laid on the membrane. By means of an automatic testing system sampling occurs at fixed points in time and analyzed with regard to their effective substance content. Hydrophilic membranes are preferably used as the membrane, with a pore size of 0.45μm (e.g. Sartorius SM 11606, Format 47, pore size 0.45μm).

**Example 1: Analagesic-rectal capsules with delayed release of the effective substance.**

Table 1

| Code | P1 | P2 | P3 | P4 | P5 | P6 |
|---|---|---|---|---|---|---|
| Substance (mg) | | | | | | |
| Paracetamol | 500 | 500 | 500 | 500 | 500 | 500 |
| Sodium carboxylmethyl starch | 0 | 100 | 200 | 300 | 400 | 500 |
| Formulations of fillings for rectal capsules with paracetamol. | | | | | | |

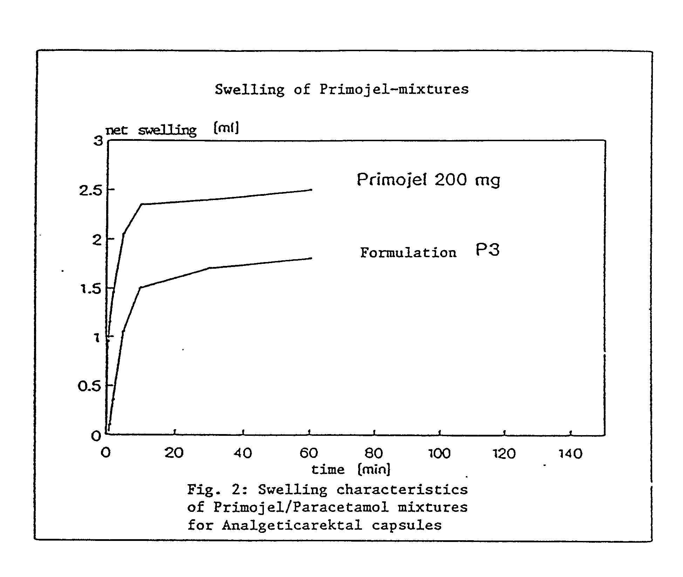

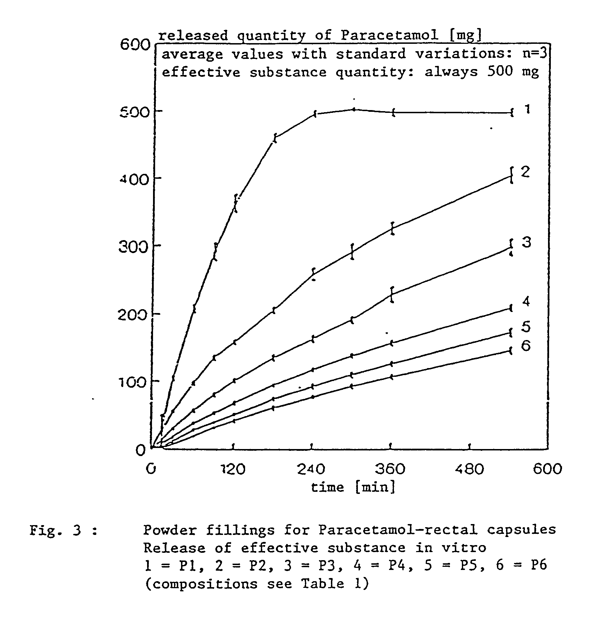

Paracetamol and sodium carboxymethyl starches are mixed for 15 minutes by means of a laboratory mixer (Turbula T2C) and subsequently filled into hard gelatine capsules of size 000. Figure 2 shows the swelling characteristics of sodium carboxymethyl starch alone as well as in the mixture P3 (see table 1); Figure 3 shows the release of the effective substances of the powder mixtures from table 1 in vitro. It can be seen directly from the results that the degree of delay can be controlled by the quantity of added swelling material and a quick release in vitro takes place without extra swelling material.

A formulation derived from P3 was tested on dogs in vivo (Race: "Holländer", Animal Hospital of the University of Zurich, average weight 17.3 kg). The rectally provided solution, as a control for a quick absorbing medicine, contained 250 mg paracetamol in 5 ml 0.9 % saline solution to which was added iodine labelled carboxymethyl starch (4%). The capsule was a traditional hard gelatine capsule (size Oel, Capsugel AG, CH-Basel) and contained 250 mg paracetamol and 200 mg sodium carboxymethyl starch (Primojel[R], Avebe, NL- Foxhol). The particle size was smaller than 125μm. In addition both formulations still contained 100 mg of an inert filler (Chelex 100, loaded with In-111).

The capsules were, in accordance with the provision of Moel et al, Stability of lubricant coats for rectal hard gelatin capsules, Acta pharma, techn. 34, 24S (1988), provided with a lubricant coat. The solution as well as the capsules were applied by means of a special applicator of Plexiglas, consisting of a cylindrical tube with an inner plunger, in order to achieve a reproducible depth of application of 5 cm. On the day of the test the dogs were either anaesthetized immediately (application of solution) or after 3 hours (application of the capsules) with a mixture of Polamivet[R], Sedalin or Combelen[R], Atropin and Pentothal[R]. After the anaesthesia blood tests were taken every hour. Blood tests were taken by means of a drip (Braunuele[R], SCC, CH-Neuhausen) and filled in Vacutainer[R] (Becton Dickinson, CH-Basel). From 4 - 7 ml blood 2 - 3.5 ml serum were yielded after coagulation in the water bath and centrifugation (3000 revolutions per minute, 10 min) and frozen at -20° C until analysis of the effective substance content.

The serum tests were mixed after immersion with 0.1 ml of a phosphate buffer pH 7.4. 1 ml of this mixture was added to an Extrelut 1 column (Merck, D-Darmstadt). After 6 minutes ethylacetate (pro analysi, Merck, D-Darmstadt) was extracted with 6 ml. The eluate was evaporated with a weak nitrogen flow at 30° C. The tests were absorbed in 400μl of the mobile phase for the HPLC and filtered through HPLC filters (0,2μm pore size, Gelman LC 13) (Skan AG, CH-Basel) in HPLC-vials. The actual HPLC analysis of the tests took place under the following conditions:

Column: Nucleosil C 18, 7μm spheric, 25 cm long, ID 4 mm (Knauer KG, CH-Belmont)

| Mobile phase: | | | |
|---|---|---|---|
| Eluant 1: | Methanol: | Water: | Phosphoric acid 85 % |
| | 20 : | 80 : | 0.3 |
| Eluant 2: | | | |
| | 75 : | 25 : | 0 |

The following gradient was used:
0 - 10 min. eluant 1, 10 - 20 min. change to eluant 2,
20 - 40 min. eluant 2, 40 - 50 min.change to eluant 1,

50 - 60 min. eluant 1.
Injection volumes : 50μl variable loop
Flow rate : 1.0 ml/min
Wavelength: 243 nm
Temperature: Room temperature

Work was carried out on an HPLC-system with an automatic test output, all were Spectra Physics, CH-Basel apparatus. The data evaluation occurred by means of on-line detection of rough data on an IBM-PC/AT and the program Labnet/Chromium station, Fa. Spectra-Physics, CH-Basel. The calculation of the concentrations was carried out by means of an external standard via the peak height. The detection-limit for paracetamol lies at 0.02μg/ml and is selective for paracetamol. The recovery rate is 88 % and is linear in the region of 0.1 to 10μg/ml. The reproducibility of 5 serum tests is typically smaller than 3 %.

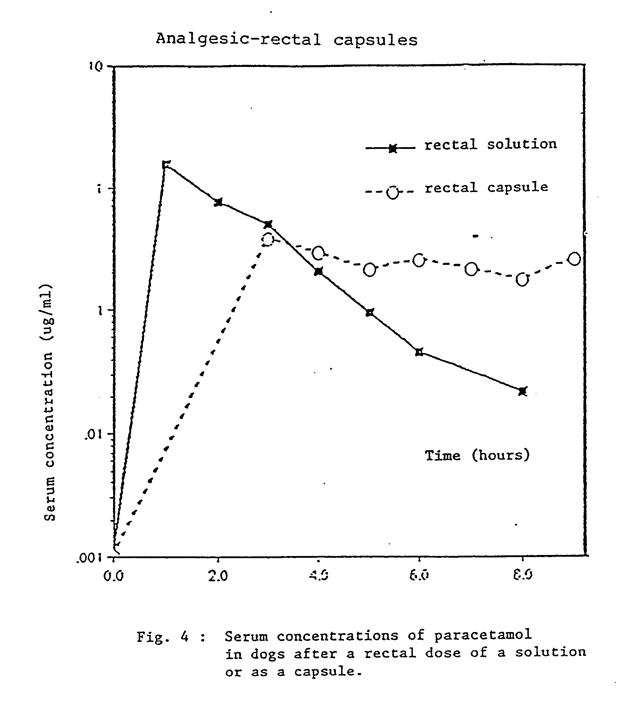

Figure 4 shows the results for two dogs with the rectal capsules (N = 2) as well as after a dose of solution (N = 1). It is clearly visible that after a dose of the solution a quick peak maximum with subsequent elimination of the effective substance according to 1st order kinetics takes place. After the dose of the capsules, on the other hand, constant serum levels of the paracetamol are achieved in the range of 3 - 9 hours. There was no absorption at all from the capsules without swelling material (not shown).

**Example 2: Anti-rheumatic effective rectal capsules with Diclofenac-Sodium.**

10g of filling, consisting of effective substance, swelling material and a further auxiliary agent as supplemental agent, were mixed for 5 minutes in a receptacle with a volumetric capacity of 50 ml in a Turbula mixer. Table 2 shows typical formulations:

Table 2

| Code | D0 | D1 | D2 | D3 | D4 |
|---|---|---|---|---|---|
|  | (mg) | (mg) | (mg) | (mg) | (mg) |
| Diclofenac Sodium | 100 | 100 | 100 | 100 | 100 |
| Primojel | - | 100 | 300 | 300 | - |
| Methocel A4M | - | - | - | - | 300 |
| Lactose | 300 | 100 | - | 100 | 100 |
| Potato starch | - | - | 100 | - | - |
| Fillings for rectal capsules with Diclofenacsodium. | | | | | |

Table 3 shows the swelling properties of the formulation D1-D4.

The net swelling is given, however, in ml, relative in each case to 100 mg of swelling material. The net swelling is calculated from the difference of the volume read at the time t = T1 minus the starting volume before adding the buffer solution, at the time t = To. With higher relative proportions of swelling material (D2 - D4) the swelling speed is increased; the final volume achieved is, however, dependent on the nature of the swelling material used. A high final volume is desirable in respect of this invention.

Table 3

| Time | Swelling vol | Code | | | |
|---|---|---|---|---|---|
| | (ml) | D1 | D2 | D3 | D4 |
| Proportion Swelling material | | 33% | 60% | 60% | 60% |
| 1.00 | | 0.18 | 0.53 | 0.53 | 0.83 |
| 2.00 | | 0.30 | 0.70 | 0.67 | 0.83 |
| 3.00 | | 0.37 | 0.83 | 0.82 | 0.90 |
| 4.00 | | 0.43 | 0.95 | 0.93 | 0.90 |
| 5.00 | | 0.48 | 1.05 | 1.02 | 0.90 |
| 10.00 | | 0.77 | 1.13 | 1.12 | 0.98 |
| 20.00 | | 0.95 | 1.18 | 1.12 | 1.00 |
| 30.00 | | 1.00 | 1.18 | 1.12 | 1.00 |
| 60.00 | | 1.00 | 1.18 | 1.13 | 1.00 |
| Swelling volume of the tested Diclofenac-fillings. | | | | | |

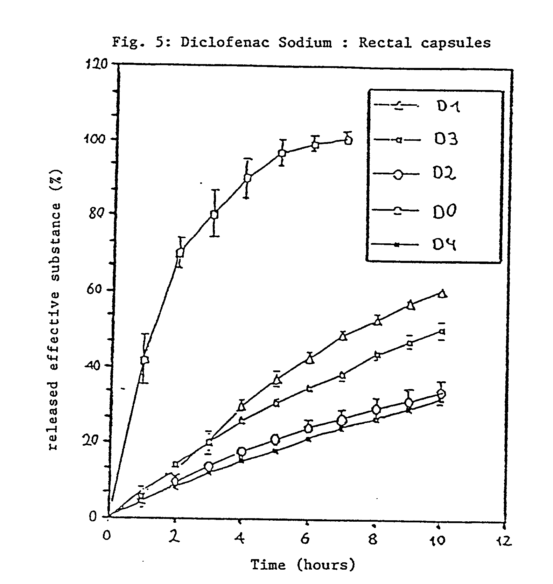

The release of the effective substance of these formulations in vitro, is determined by means of the method described above, is shown in figure 5. Compared with the capsules without swelling material (formulation DO) the addition of sodium carboxymethyl starch results in a delayed release of the effective substance, wherein the effect of delay rises with an increase in the concentration of swelling material (formulation D1 and D3). The addition of a further swelling material (potato starch) results in an additive effect on the delay (formulation D2). A similar retarding effect can, however, also be achieved by using a swelling material of another characteristic (formulation D4).

## Example 3: Disinfecting vaginal capsules PVP-IODINE.

Table 4

| Formulations | V1 | V2 | V3 | V4 | V5 |
|---|---|---|---|---|---|
| | (mg) | (mg) | (mg) | (mg) | (mg) |
| PVP-iodine 7/12 | 250 | 250 | 250 | 250 | 250 |
| Sodium citrate | 69 | 69 | 69 | 35 | 35 |
| Citric acid | 34 | 34 | 34 | 17 | 17 |
| Sodium carboxylmethyl starch | - | 147 | - | - | 198.5 |
| Methylhydroxypropyl cellulose | - | - | 147 | 198.5 | - |
| Lactose | 147 | - | - | - | - |
| Fillings for vaginal capsules with PVP-IODINE. | | | | | |

Iodine has for a long time been known as a disinfecting means. PVP-Iodine is an aggregate of polyvinyl pyrrolidone and iodine. In the present tests the quality PVP-Iodine 17/12 was used, as marketed by BASF AG, D-Ludwigshafen. Other qualities can also be used, e.g. PVP-Iodine 17/12 M or PVP-Iodine 30/06, 30/06 M from BASF or other manufacturers.

The amount of effective substance released in vitro was determined with the model shown in figure 1, temperature 37 °C, speed of revolutions 150 R/min. A citrate buffer pH 4.4 of the following composition served as releasing agent:

8

| Citric acid (free of water) | 21.04 g |
| Sodium hydroxide solution 1 N | 200.0 g |
| Distilled water ad | 1000 ml |

All tests were carried out three times. In each case 3ml of a 0.1 sodium thiosulphate solution were added to 300 ml citrate buffer pH 4.4 (in 500 ml beakers). The capsule is placed in the cylinder on the membrane. Because of the thiosulphate contained in the solution the iodine, which is released from the formulation, is transformed directly into iodide. At regular intervals 10 ml releasing medium were removed and the remaining sodium thiosulphate determined with 0.001N iodine solution, wherein 1 ml of 1% starch solution served as indicator.

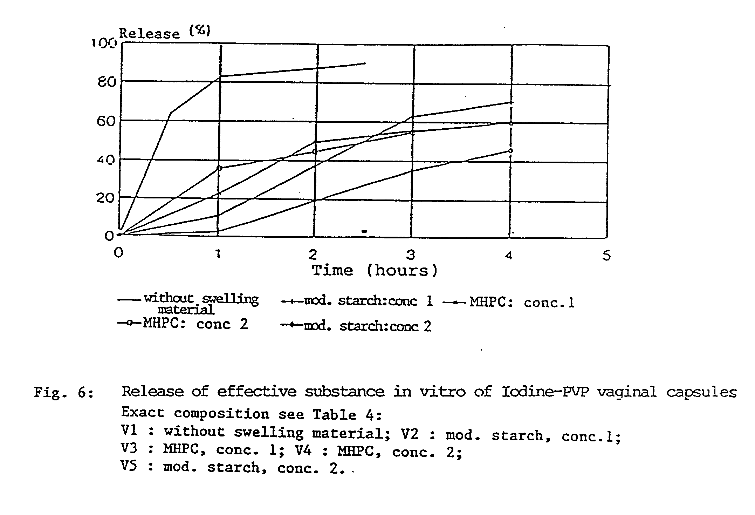

The results are represented in figure 6. Capsules which contain no swelling material and only contain lactose as filler (formulation 1) quickly release the PVP-Iodine after a short lag-time, due to the disintegration of the capsule.

Methylhydroxypropyj cellulose (Form 3.4) as well as sodium carboxymethyl starch (Form 2.5) lead to a considerable delay of the effective substance discharge. Due to the use of the slow-swelling methylhydroxypropyl cellulose a higher release is achieved initially since the delaying effect only manifests itself if the swelling material swells out to a gel. With sodium carboxymethyl starch one very quickly obtains a gel, so that the release from the beginning is greatly delayed and no so-called "burst effect" occurs. Since such a burst effect is desired with some medicines and with others is less desirable, the release profile can be correspondingly controlled by selecting the swelling material. The speed of the effective substance release can be controlled with both forms by the proportion of swelling material, wherein this effect is more pronounced with sodium carboxymethyl starch.

## Example 4: Rectal capsules with propranolol-hydrochloride

1 kg propranolol-hydrochloride (BP 1980) was mixed with 10 kg sodium carboxymethyl starch in a V-mixer. 10 kg lactose as filler is added to this mixture. 420 mg are filled in each case into hard gelatine capsules of Oel size, corresponding to a single dose of 20 mg propranolol-HCl.

The effective substance release in vitro is determined with the standard measuring system (figure 1) with the following conditions:
Testing medium : phosphate buffer pH 7.4, isotonic and isohydric, ion strength = 0.25; volume 750 ml, Temperature 37.0 ° C, number of revolutions 150 revolutions/min, test volume 10 ml.

At the beginning of the test, as well as after 15, 30, 60, 90, 120, 180, 240, 360 and 540 minutes, tests are carried out in which the content, possibly after suitable thinning with the testing medium, is measured in a spectrophotometric manner at 290 nm.

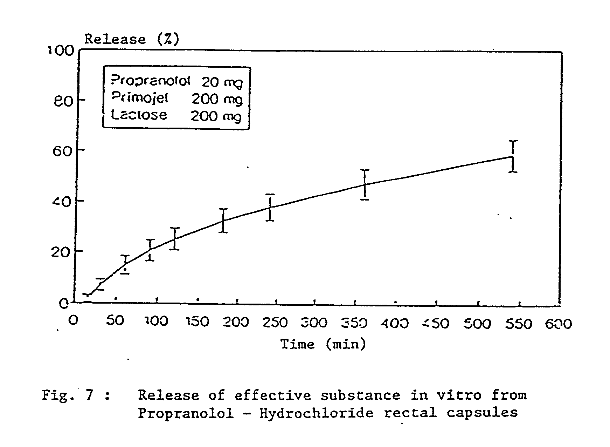

The results of 3 analyses respectively are represented in figure 7. After a lag-time of approx. 10 minutes, determined by the disintegration of the capsule, a delayed discharge of the lightly soluble effective substance propranol occurs over several hours.

## Example 5 : Rectal capsules with insulin.

Capsules with a dosage of 35 I.E. per capsule were manufactured, wherein the following method was used (quantity relative to the dosing in the animal test).

100 mg Aerosil 200 (Degussa Ag, D-Hamburg) are mixed with 0.7 ml insulin solution (Insulin Actrapid[R] Novo HM Penfil, 100 I.E./ml, diluted with 0.9% sterile saline solution with a ratio 1:1). The moist powder is dried for 2 hours at room temperature in the vacuum drier. After crushing in a mortar the citric acid and, depending on the formulation, sodium carboxymethyl starch is added and mixed for 10 minutes. This mixture is either filled in capsules of the size Oel or processed according to the cream melting method into a suppository.

Table 5 shows the compositions of the tested capsules; the composition of the suppositories corresponds to the formulation F, wherein, instead of filling into a capsule, 1.8 g Witepsol H 15 was used as suppository mass. The intravenous doses of insulin (0.05 - 0.2 I.E./kg) as well as rectal doses of insulin in solution, with and without the addition of citric acid for pH adjustment, served as controls.

The bioavailability of the different preparations was tested on white New Zealand rabbits with an

9

average weight of 3.5 ± 0.2 kg. The animals were kept in an air-conditioned room at 23° C celsius with a relative air humidity of 40 - 50 %. A bright-dark rhythm of 12 hours was maintained. The animals received standardized feed during the whole duration of the tests. 5 days before the respective test the animals received no solid feed, but only glucose solution respectively a modified Ringer solution. In the morning of the day of the test the animals were put into a special test box which limited movement. The blood extraction was carried out by means of a lancet from the right ear vein (32μl test volume). After the following times blood tests were taken : 15 min before application (100% value) as well as 0.5, 1.0, 2.0, 3.0, 4.0 and 6.0 hours after the application. The blood sugar level of these tests was determined by means of gluco test strips and a Reflotron[R] (Fa Boehringer, Mannheim GmbH, D-Mannheim). The blood sugar value obtained before the application of the pharmaceutical formulations, respectively solutions, was defined as the starting value (100%). The blood sugar values determined at the respectively measuring times are given as percentage fractions of this starting value. The rectal formulations were applied with the aid of a special application tube, in order to create reproducible conditions for the different formulations.

The following values were established: maximum effect corresponding to a maximum lowering of the blood sugar level in % (Emax); area under the effect/time curve (AUC); absolute bioavailability (Fabs) as quotient of the rectal forms compared to the intravenous application as well as relative bioavailability (Frel) of the rectal forms compared to the rectal dose of a solution with the dosage 10 I.E./kg. For assessing the delaying effect of the different formulations the mean residence times (MRT) after the dose of the different formulations were calculated, wherein those respective dosages were used, which were lying approximately in the linear region of the dosage effective curves.

From table 6 it can be seen that insulin is 4 times more available when supplied in the form of rectal capsules, if one compares the same dosages of capsules and solutions (10 I.E./kg). If one compares the capsules with those formulations, which contain the same quantity of citric acid, but no swelling material and are provided as conventional rectal form of administration e.g. as solution or as suppository, then the bioavailability of the insulin from the capsules is roughly double as high as that from the usual formulations of administration.

From table 6 it can be seen that when using rectal capsules, due to the use of a swelling material (Aerosil), a clear extension of the mean residence time occurs, compared with a suppository or solution. With a dose of suppositories, on the other hand, there was no delay; the average periods for suppositories and rectally applied solution do not vary significantly (p = 0.05; dosage 10.I.E./kg, see table 6). By adding a further swelling material an even greater delay can occur (addition of sodium carboxymethyl starch in addition to the Aerosil already contained in the formulation).

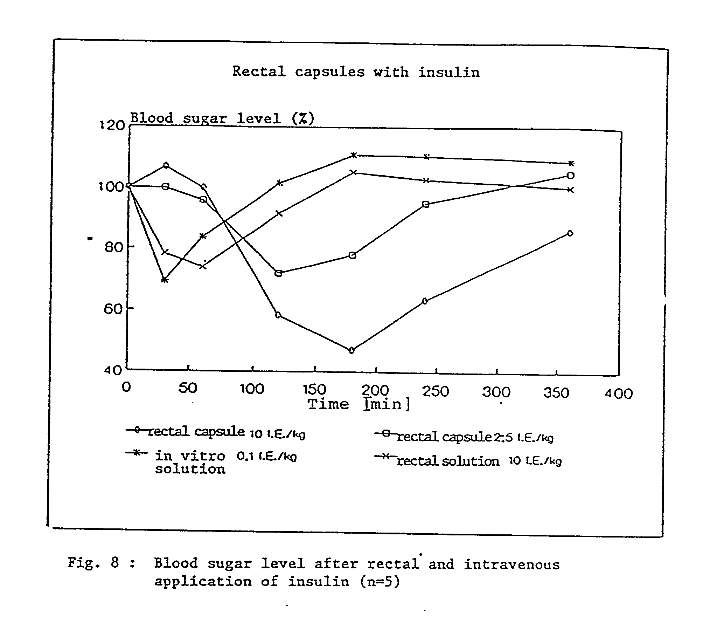

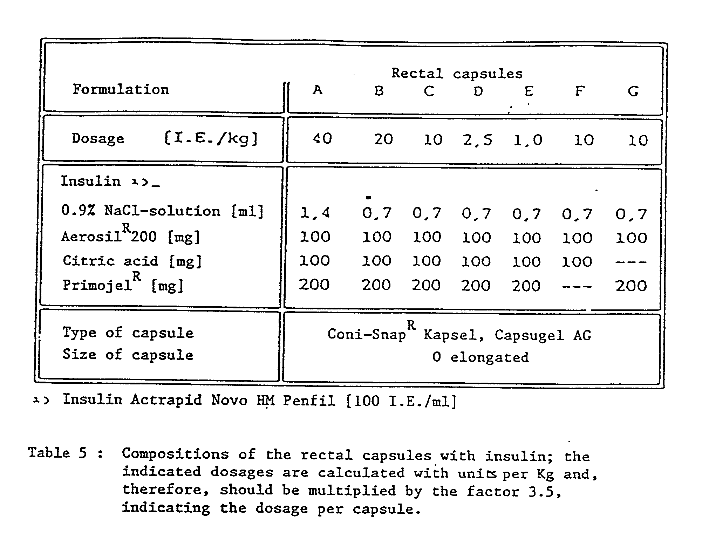

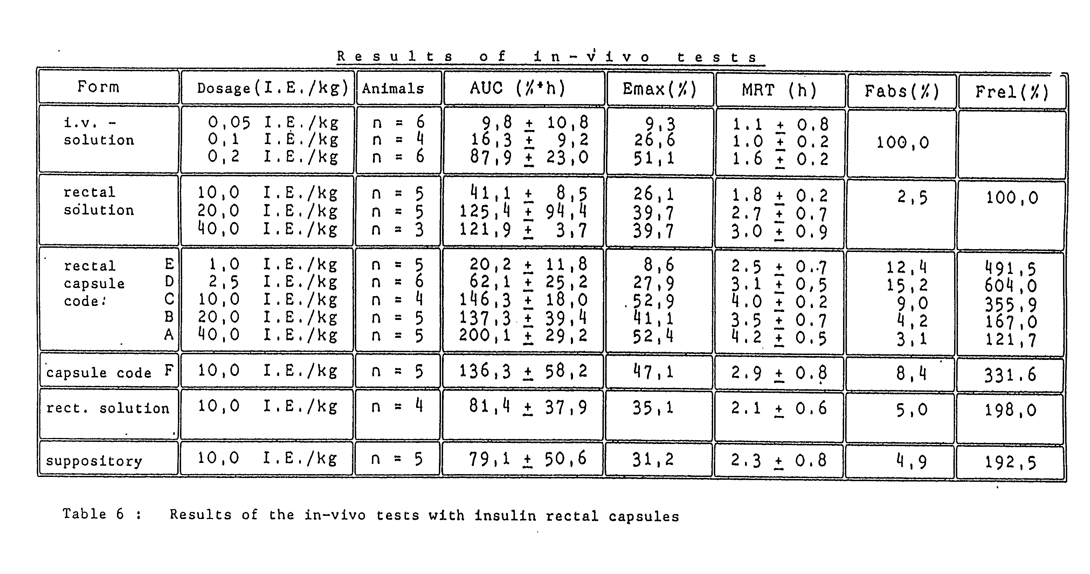

In figure 8, the glucose concentrations in the blood after an intravenous dose of a solution, a rectal dose of a solution and rectal dose of a capsule are represented as an illustration of the delaying effect after a dose of the capsules with swelling materials. After a dose of a capsule the effect of the lowering of the blood sugar is maintained substantially longer than with a rectally applied solution of the same concentration or an intravenously applied solution, which generated the same maximum effect (Emax).

## Claims

1. A composition for rectal or vaginal application of medicines, characterised in that the composition is substantially free of fat and contains, together with the effective substance or effective substances and possibly further carrier substances, at least one hydrocolloid, which, through interaction with the local body fluid, effects a controlled release of the effective substance.

2. A composition according to claim 1, characterised in that it is substantially free of water.

3. A composition according to one of claims 1 or 2, characterised in that it is present as granules or powder and has a maximum particle size of 250μm and preferably 150μm.

4. A composition according to one of claims 1 - 3, characterised in that it is manufactured by simple mixture of the components.

5. A composition according to one of claims 1 - 4, characterised in that it contains a pharmaceutically effective substance which has, in systemic or local therapy, a rectal or vaginal application.

6. A composition according to claim 5, characterised in that the effective substance is solid at room temperature or, by means of absorbtion on a carrier material, is transformed into a solid form.

7. A composition according to one of claims 1 - 6, characterised in that the therapeutically effective substance is a polypeptide of low to medium molecular weight, preferably vasopressin, calcitonin, insulin or interleukine; an anti-rheumatic agent preferably Diclofenac-sodium; an analgesic preferably paracetamol, morphine or morphine equivalent; an anti-emetic agent preferably metoclopramide; an anti-mycotic preferably clotrimazole or amphotericin-B; an anti-infectant preferably povidone-iodine; a hormone preferably estradiol, prednisolone or a mixture of such substances.

8. A composition according to one of claims 1 - 7, characterised in that it contains in addition, a fat and/or a polyethylene glycol in pulverized or powdery form, in a concentration of up to 30 weight %, preferably in a concentration below 20 % by weight and particularly below 10 % by weight, relative to the total weight of the composition.

9. A composition according to one of claims 1 - 8, characterised in that it contains the hydrocolloid in a concentration of at least 5 % by weight, preferably at least 10 % by weight and preferably at least 25 % by weight.

10. A composition according to one of claims 1 - 9, characterised in that this contains the hydrocolloid in a concentration of 5 - 99.9 % by weight, preferably of 10 - 95 % by weight, and particularly of 40 - 90 % by weight relative to the total weight of the composition.

11. A composition according to one of claims 1 - 10, characterised in that the viscosity of a 2 % aqueous preparation of hydrocolloid, measured by means of an Ubbelohde capillary viscometer or Brookfield viscometer at 20° C, lies below 5000 cps, preferably below 1000 cps.

12. A composition according to one of claims 1 - 11, characterised in that this contains, together with the effective substance and the hydrocolloid, a flow regulating agent, a lubricant, a filler, a carrier substance or a wetting agent or a mixture of such materials and possibly further auxiliary agents which improve stability, processibility or bioavailability.

13. A composition according to one of claims 1 -12, characterised in that the following ingedients are present in percentage parts by weight:

| (a) effective substance | 0.0001% - 80 % |
| (b) hydrocolloid | 20 % - 90 % |
| (c) flow regulating agent | 0.2 % - 4 % |
| (d) lubricant | 0.5 % - 4 % |
| (e) filler | 0 % - 70 % |
| (f) carrier substances | 0 % - 10 % |
| (g) wetting agent | 0.1 % - 5 % |

14. A method for producing a composition according to one of claims 1 - 13, characterised in that the individual components are mixed together in an intensively mechanical manner.

15. Capsules, preferably capsules with a hard casing, in particular those of gelatine or starch, for rectal or vaginal application, which contain a composition in accordance with the invention, according to one of claims 1 to 13.

16. Two-part hard gelatine capsules according to claim 15 manufactured by means of the dipping method, which are coated with a lubricant coat and possibly additionally sealed and/or provided with a band, which covers the cap edge and a bordering part of the outer wall of the container part

17. Capsules according to claim 15, which are injection moulded.

18. A composition according to anyone of the claims 1 to 13, characterized in that said composition is used in rectal or vaginal application.

Fig. 1 : Measuring system for determining the release of the effective substance
in vitro
Left side : cylinder with holder for membranes
Right side : schematic representation of membrane and sealing rings.
The plunger can be used to achieve a reproducible thickness of layer,
but is not necessary for the examination of capsules.

Fig. 2: Swelling characteristics
of Primojel/Paracetamol mixtures
for Analgeticarektal capsules

Fig. 3 :    Powder fillings for Paracetamol-rectal capsules
Release of effective substance in vitro
1 = P1, 2 = P2, 3 = P3, 4 = P4, 5 = P5, 6 = P6
(compositions see Table 1)

### Analgesic-rectal capsules

Fig. 4 : Serum concentrations of paracetamol
in dogs after a rectal dose of a solution
or as a capsule.

Fig. 5: Diclofenac Sodium : Rectal capsules

Fig. 6: Release of effective substance in vitro of Iodine-PVP vaginal capsules
Exact composition see Table 4:
V1 : without swelling material; V2 : mod. starch, conc.1;
V3 : MHPC, conc. 1; V4 : MHPC, conc. 2;
V5 : mod. starch, conc. 2.

Fig. 7 :   Release of effective substance in vitro from
Propranolol – Hydrochloride rectal capsules

Fig. 8 : Blood sugar level after rectal and intravenous
application of insulin (n=5)

| Formulation | Rectal capsules | | | | | | |
|---|---|---|---|---|---|---|---|
| | A | B | C | D | E | F | G |
| Dosage        [I.E./kg] | 40 | 20 | 10 | 2,5 | 1,0 | 10 | 10 |
| Insulin ˣ⁾_ | | | | | | | |
| 0.9% NaCl-solution [ml] | 1,4 | 0,7 | 0,7 | 0,7 | 0,7 | 0,7 | 0,7 |
| Aerosil$^R$200 [mg] | 100 | 100 | 100 | 100 | 100 | 100 | 100 |
| Citric acid [mg] | 100 | 100 | 100 | 100 | 100 | 100 | --- |
| Primojel$^R$ [mg] | 200 | 200 | 200 | 200 | 200 | --- | 200 |
| Type of capsule | Coni-Snap$^R$ Kapsel, Capsugel AG | | | | | | |
| Size of capsule | 0 elongated | | | | | | |

ˣ⁾ Insulin Actrapid Novo HM Penfil [100 I.E./ml]

Table 5 :   Compositions of the rectal capsules with insulin; the indicated dosages are calculated with units per Kg and, therefore, should be multiplied by the factor 3.5, indicating the dosage per capsule.

| Form | Dosage(I.E./kg) | Animals | AUC (%*h) | Emax(%) | MRT (h) | Fabs(%) | Frel(%) |
|---|---|---|---|---|---|---|---|
| i.v. - solution | 0,05 I.E./kg<br>0,1 I.E./kg<br>0,2 I.E./kg | n = 6<br>n = 4<br>n = 6 | 9,8 ± 10,8<br>16,3 ± 9,2<br>87,9 ± 23,0 | 9,3<br>26,6<br>51,1 | 1.1 ± 0.8<br>1.0 ± 0.2<br>1.6 ± 0.2 | 100,0 | |
| rectal solution | 10,0 I.E./kg<br>20,0 I.E./kg<br>40,0 I.E./kg | n = 5<br>n = 5<br>n = 3 | 41,1 ± 8,5<br>125,4 ± 94,4<br>121,9 ± 3,7 | 26,1<br>39,7<br>39,7 | 1.8 ± 0.2<br>2.7 ± 0.7<br>3.0 ± 0.9 | 2,5 | 100,0 |
| rectal E<br>capsule D<br>code: C<br>B<br>A | 1,0 I.E./kg<br>2,5 I.E./kg<br>10,0 I.E./kg<br>20,0 I.E./kg<br>40,0 I.E./kg | n = 5<br>n = 6<br>n = 4<br>n = 5<br>n = 5 | 20,2 ± 11,8<br>62,1 ± 25,2<br>146,3 ± 18,0<br>137,3 ± 39,4<br>200,1 ± 29,2 | 8,6<br>27,9<br>52,9<br>41,1<br>52,4 | 2.5 ± 0.7<br>3.1 ± 0,5<br>4.0 ± 0.2<br>3.5 ± 0.7<br>4.2 ± 0.5 | 12,4<br>15,2<br>9,0<br>4,2<br>3,1 | 491,5<br>604,0<br>355,9<br>167,0<br>121,7 |
| capsule code F | 10,0 I.E./kg | n = 5 | 136,3 ± 58,2 | 47,1 | 2.9 ± 0.8 | 8,4 | 331.6 |
| rect. solution | 10,0 I.E./kg | n = 4 | 81,4 ± 37,9 | 35,1 | 2.1 ± 0.6 | 5,0 | 198,0 |
| suppository | 10,0 I.E./kg | n = 5 | 79,1 ± 50,6 | 31,2 | 2.3 ± 0.8 | 4,9 | 192,5 |

Table 6 :   Results of the in-vivo tests with insulin rectal capsules

EP 0 391 852 A2